# EUROPEAN PATENT SPECIFICATION

(11) **EP 1 667 594 B1**
(45) Date of publication and mention of the grant of the patent: **02.02.2011**
(21) Application number: 04768290.1
(22) Date of filing: 31.08.2004
(51) Int. Cl.: A61B 17/86, A61B 17/17

(54) **Jig for insertion of a bone fixing device**
Bohrlehre zum Einführen einer Knochenfixiervorrichtung
Gabarit d'insertion d'un dispositif de fixation d'os

(30) Priority: 30.08.2003 GB 0320375
(43) Date of publication of application: 14.06.2006
(73) Proprietor: GRAMPIAN HEALTH BOARD, Aberdeen AB15 6RE (GB); The Robert Gordon University, Aberdeen, AB10 1FR (GB)
(72) Inventor: JOHNSTONE, Alan John, Bieldside, Aberdeen AB15 9AD (GB)
(74) Representative: Brown, Deborah Frances
(86) International application number: PCT/GB2004/003742
(87) International publication number: WO 2005/020831

(56) References cited:
- WO-A-99/18873
- WO-A-03/072962
- WO-A-03/092515
- DE-A1- 19 751 284
- GB-A- 2 147 504
- US-A- 4 175 555
- US-A- 5 643 273
- US-A1- 2002 165 551

## Description

The present invention concerns an apparatus for treating a fracture in a bone, especially but not exclusively for treating a wrist fracture.

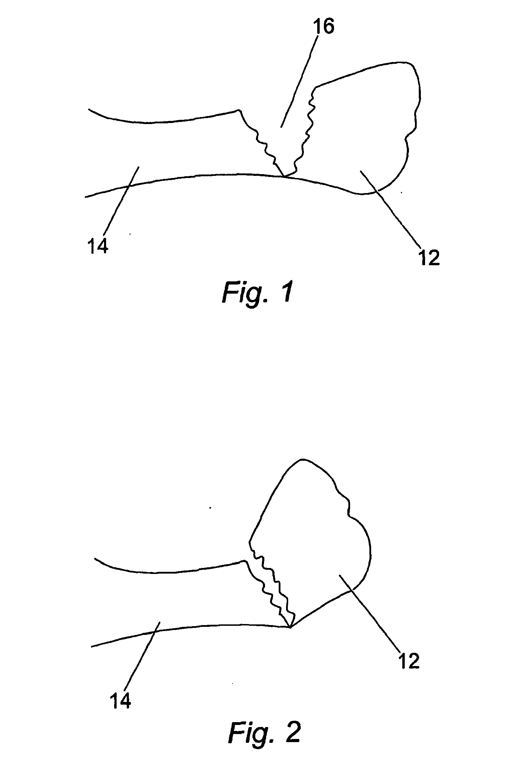

When a wrist bone breaks, it often fractures into many fragments on the dorsal side of the wrist, whilst leaving a fairly clean break on the lower side. A typical broken wrist is shown in Fig 1. Distal bone portion 12 needs to be held fixed relative to proximal bone portion 14 in the position shown in Fig 1, so that the bone portions 12, 14 do not heal in the position shown in Fig 2. Further bone fragments generally float in the space designated 16. These will fuse with bone portions 12, 14 as the fracture heals.

One method of treating a wrist fracture involves attaching a metal frame to the outside of the wrist, spanning the wrist joint. Pins typically extend from the frame into the bone portions on each side of the fracture. This can be an invasive method, and can immobilise the joint for a long period, resulting in a very stiff joint once the fracture has healed. A further alternative method involves fixing the fracture using plates and screws. All of these methods are very invasive, in some cases totally preventing movement of the joint and are likely to be very inconvenient to the patient.

This invention relates to a jig to aid insertion of a bone fixing device into a bone to treat a fracture. This invention relates especially but not exclusively to a jig which aids the relative alignment of at least two bone screws.

Bones often fracture into a plurality of bone fragments. In this case, it is helpful to insert two screws along different line of insertion, so as to span most of or all of the fragments. It is highly undesirable if these two screws collide with each other, because this could prevent the second screw from being fully inserted into the bone, and could also force the first screw out of position. If this happens, the second screw would have to be removed and a new hole made.

Therefore, to avoid this problem, the second screw is typically inserted at a safe distance away from the first screw so that there is a negligible chance of a collision. However, once the screws have been inserted, the only support keeping the screw in the bone is friction between the bone and the screw threads. In patients with fragile crumbly bone, e.g. some elderly patients, this friction may not be sufficient, and the bone screws may work themselves loose. This could further damage the bone and additional surgery may be required.

According to the invention there is provided an assembly according to claim 1.

Provision of the jig allows the second line of insertion to be defined very accurately with respect to the first line of insertion. Because the lines of insertion are in parallel planes, they can never intersect, and the possibility of bone fixing devices colliding with each other is reduced.

Optionally, more than two lines of insertion could be defined by the jig.

Typically, the bone fixing devices are at least partially threaded. Preferably, the bone fixing devices comprises bone screws.

Preferably, the parallel planes are relatively close to one another. The parallel planes have a minimum separation equal to the diameter of the bone fixing device. If the fixing device comprises a threaded fixing device, e.g. a bone screw, the parallel planes have a minimum separation equal to the outer thread diameter of the bone fixing device. Such embodiments can ensure that the bone fixing devices will not collide.

Some such embodiments can provide that the bone fixing devices interact, thus providing an additional connection to each other, which helps to hold the bone fixing devices in the bone. "Interact" is used in the sense that the bone fixing devices can touch and can lean against each other, one fixing device effectively buttressing and supporting the other.

Preferably, the orientation of the second line of insertion is variable relative to the orientation of the first line of insertion. Preferably, the second line of insertion is translatable relative to the first line of insertion.

"Translation" is used throughout the specification to mean not only movement along a straight line, but also movement along an arcuate path. "Translational movement" is used as a way of differentiating between rotational movement about an axis.

Preferably, the jig includes an arm that extends in a lateral direction with respect to at least one of the lines of insertion.

Preferably, the arm is arcuate.

Preferably, at least a part of the arm has a planar surface that is parallel to the parallel planes of the lines of insertion. Optionally, at least a part of the arm has a planar surface that is co-planar with one of the parallel planes of the lines of insertion.

The first and second lines of insertion are defined by respective first and second guide means coupled to the arm. Typically, the first and second guide means have bores that serve to align the path of the guide wire or bone fixing device with the desired line of insertion. In some embodiments, the guide means may include at least one guide sleeve received in the bore of the guide means and the guide sleeve can be slidably and/or rotatably mounted in the bore so that it is releasably coupled to the arm. Typically, the arm is rotatably mounted relative to the guide means so that the arm can pivot around the axis of the guide means.

A guide means can be anything which can guide the path of a guide wire and/or a bone fixing device. Typically, the first and second guide means can include first and second guide sleeves. Optionally, each of the first and second guide means comprises a plurality of concentric sleeves. However, in more basic embodiments, an apertured section of the arm can suffice, and separate guide sleeves are not necessary.

Typically, the first and second guide means are adapted to receive guide wires and/or bone fixing devices. The invention is not limited to the use of guide wires because in some embodiments, the first and second guide means can directly guide the bone fixing devices into the bone.

Optionally, the jig includes a spacer adapted to space the parallel planes of the lines of insertion apart from each other.

Typically, the spacer is a separate component which is coupled to the arm.

Optionally, the spacer comprises a block having a guide bore adapted to receive a guide means. Preferably, the guide means can be slidably mounted in the guide bore, and can optionally rotate relative thereto.

Optionally, the spacer has an attachment bore and the spacer is coupled to the arm by an attachment device inserted through the attachment bore in the spacer and also into/through the arm. Preferably, the spacer is rotatably mounted on the arm. Typically, the attachment device comprises a bolt and a nut; the bolt and nut connection can be loosened to allow rotation of the spacer relative to the arm. After selection of the required angle, the nut can be tightened to fix the rotational position of the spacer relative to the arm.

Optionally, an elongate aperture is provided in the arm, and the spacer is moveable along the elongate aperture. If the attachment device is a bolt and a nut, the bolt can be loosened to allow translation of the spacer relative to the arm using the elongate aperture, and then tightened again to restrict further translational movement once a position has been selected.

Optionally, the arm may be extendible to allow relative translational movement of the first and second guide means.

Typically, the position of one of the guide means is defined by the arm and the position of the other guide means is defined by the spacer, such that rotating the spacer relative to the arm varies the orientation of the second guide means relative to the first guide means, and translating the spacer along the arm varies the distance between the first and second guide means.

The spacer is not necessarily a separate component. Alternatively, the spacer could comprise a part of the arm. For example, the arm could comprise a first portion and a second portion having parallel planar surfaces, wherein the second portion is stepped relative to the first portion. In such embodiments, the step in the arm can function as a spacer to space the two parallel surfaces of the first and second parts of the arm from each other, and these parallel surfaces can define the parallel planes of the first and second lines of insertion.

In such embodiments, the position of the second guide means may be fixed in angular orientation and/or translational position relative to the first guide means (apart from any optional slidable mounting to the arm). For example, such an embodiment may be made by attaching a first guide means to one end of the arm and a second guide means to the other end of the arm on the other side of the stepped portion. However, in preferred embodiments, at least one of the guide means is rotatably mounted on the arm. This could be achieved, for example, by hingedly mounting the second guide means to the arm. In this way, the relative angles of insertion of the two bone fixing devices can be selected.

In some embodiments, a stepped arm and a separate spacer component can both be used in conjunction to provide the two parallel planes of the lines of insertion.

In all embodiments, the arm may optionally have an elongate slot to enable relative translational movement of the first and second guide means.

In all embodiments, the arm may optionally be extendible to enable relative translational movement of the first and second guide means.

Embodiments of the invention will now be described, by way of example only, and with reference to the following drawings, in which the described bone fixing devices are only embodiments of the invention as parts of assemblies according to the claim 1 :-
Fig 1 shows a side view of a broken wrist with bone portions in the correct alignment to heal;
Fig 2 shows a side view of a broken wrist, with bone portions incorrectly aligned;
Fig 3 shows a cross-section of two bone portions with a guide wire inserted through both bone portions;
Fig 4 shows a cross-section of the bone portions of Fig 3, with a fixing device inserted through the portions;
Fig 5 shows a side view of a further alternative embodiment of a fixing device having threads of varying pitch;
Fig 6 shows a side view of a further alternative embodiment, having threads of different pitches and two portions of different diameters;
Figs 7a and 7b show a further embodiment, having steeply sloping screw threads of varying pitch;
Fig 8 shows a side view of a further embodiment, having steeply sloping screw threads of varying pitch and a varying diameter;
Figs 9a and 9b show sectional views of a further embodiment, having rectangular threads of varying pitch;
Fig 10 shows a sectional view of a further embodiment, having rectangular threads of varying pitch and varying diameter;
Figs 11a and 11b show a further embodiment, having perpendicular screw threads in each axially-facing direction, the threads having a varying pitch;
Fig 12 shows a side view of a further embodiment, having perpendicular screw threads in each axially-facing direction of varying pitch and varying outer diameter;
Fig 13 shows a cross-sectional view of a jig of the present invention being used to insert guide wires into a bone;
Fig 14 shows a cross-section view of part of a second embodiment of the invention;
Fig 15 shows a schematic view from above of two fixing devices inserted along respective lines of insertion I1, I2, wherein the distance apart of the parallel planes of the lines of insertion equals the diameter of the fixing device;
Fig 16 shows a schematic view similar to Fig 15, wherein the fixing devices are threaded and the spacing of the parallel planes equals the outer (thread) diameter of the fixing devices;
Fig 17 shows a schematic top view of a modified jig having a stepped arm;
Fig 18 shows a schematic top view of a modification to the Fig 17 stepped arm jig;
Fig 19 shows a schematic top view of an alternative jig having two spacers and a non-stepped arm;
Fig 20 shows a sectional view of a further embodiment, having steeply sloping screw threads of consistent pitch;
Fig 21 shows a sectional view of a further embodiment, having rectangular threads of consistent pitch; and
Fig 22 shows a sectional view of a further embodiment, having perpendicular screw threads in each axially-facing direction.

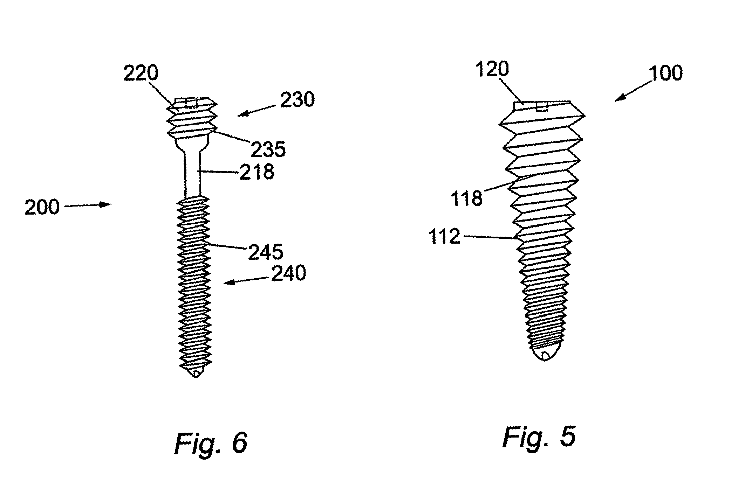

Referring now to the drawings, Fig 5 shows a screw 100 having a head 120, a root 118 and screw threads 112 all the way along its length. The screw 100 is tapered, i.e. the diameter of root 118 and the outer diameter of screw threads 112 typically both increase uniformly towards the head 120. Such tapering can allow the screw 100 to be more easily inserted into a pilot hole; in some cases, a pilot hole may not even be required. Furthermore, the increasing diameter of the root 118 and the threads 112 towards the head end of screw 100 will urge the root 118 against previously uncut portions of bone, and the threads will cut into new bone which was not previously cut by the smaller diameter threads, both of which will secure the screw 100 more firmly into the bone than could be achieved with other designs.

The pitch of screw threads 112 increases uniformly towards the head 120. The pitch of the threads is typically in the range 1mm to 2mm at the leading (tip) end, and 2mm to 4mm at the trailing (head) end. The screw 100 will typically cause a relative distraction of bone portions 12, 14 of 1mm to 2mm per rotation.

The head 120 is flush with the outer surface of root 118 so that the head 120 is an axial continuation of the body of the screw 100 and it does not project radially outward of the root 118. Alternative embodiments may have an outwardly projecting head. In this example, screw 100 is cannulated to allow insertion over a guide wire (not shown). However, non-cannulated embodiments can also be used. The head 120 may have an aperture for insertion of an Allen key or other torque tool. The screw 100 preferably has a self-drilling, self-tapping tip.

Although screw 100 shown here has screw threads 112 extending along the whole of its length, alternative embodiments (not shown) may have screw threads only at particular portions, and not at others, for example, at leading and trailing ends, with a mid-portion remaining unthreaded.

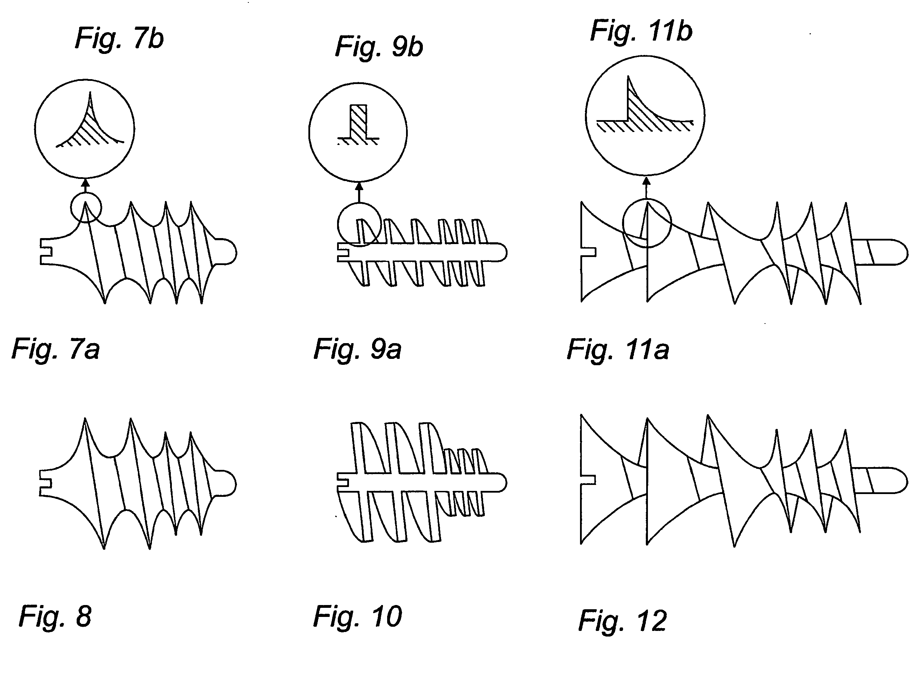

Alternatively, the root diameter could be constant, and the outer thread diameter alone could increase towards the head 40 (examples of such embodiments are found in Figs 8, 10 and 12, described below).

Fig 6 shows a screw 200 (having a root 218 and a head 220), which is similar to screw 100, in that the root 218 and threads vary in diameter. However, instead of varying uniformly, screw 200 is divided into two portions of different diameter: a trailing portion 230 having screw threads 235 and a leading portion 240 having screw threads 245. Both the diameter of the root 218 and the outer diameter of the screw threads 235, 245 are different between each of the two portions, but are uniform within each portion 230, 240. The trailing portion 230 has a larger diameter root (of around 2mm to 4mm) and a larger outer diameter of screw thread (of around 3mm to 6mm) than the leading portion 240 (3mm to 5mm and 4mm to 7mm for root and thread respectively). Threads 230 have a constant pitch, as do threads 240, but the pitch of screw threads 230 is approximately 2-4mm, which is greater than the pitch of screw threads 240 at approximately 1-2mm. Thus, the diameter of the root and the threads, and the thread pitch varies in the same way as for screw 100, except that the variation is abrupt between the two portions 230, 240, instead of continuous. As with screw 100, embodiments of screw 200 may be cannulated; may have a self-drilling, self-tapping tip; may have a non-threaded mid-portion; and may have a head which does not project outwardly of its root. The arrangement shown in Fig. 6 could of course be modified so that the sides of the root of the screw 200 are parallel so that the diameter of the root is uniform for the length of the screw 200. Such a modification could optionally have the variations in the diameter and pitch of the threads that are shown in Fig. 6.

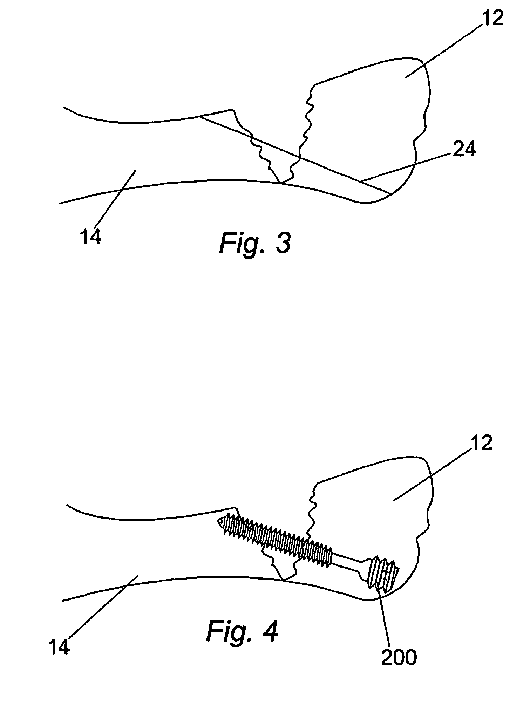

In use, the bone portions 12, 14 are first aligned into the correct position shown in Fig 1. Next, a K-wire 24 is typically inserted into the bone to span the fracture, with opposite ends of the K-wire 24 engaging respective bone portions 12, 14. An image intensifier (apparatus which produces low intensity x-rays) is typically used to check that the K-wire 24 is correctly positioned, as shown in Fig 3. The length of protruding guide wire is measured and deducted from the known length of the wire to indicate the correct screw length.

Next, the screw 100, 200 is inserted over the K-wire 24 and screwed into the bone portions 12, 14, e.g. by using an Allen key, as shown in Fig 4. If the screw 100,200 is not self-drilling and/or self-tapping, then a hole would have to be drilled and/or tapped before inserting the screw 100, 200 e.g. with a cannulated drill inserted over K-wire 24. A jig is optionally used to align the screw 100, 200 and/or the K-wire, and/or any drill used.

The leading end (tip end) of the screw 100,200 engages bone portion 14 and the trailing end (head end) engages bone portion 12. Since the head 120,220 is merely an extension of the body of screw 100,200 and it does not protrude radially outward from the root 118, the head 120,220 can be driven completely inside bone portion 12 along with the rest of screw 100,200 (see Fig 4), as opposed to complete insertion being prevented by a radially-extending head abutting against the exterior surface of cortical bone portion 12.

If a screw having a radially-extending head were to be fully inserted into bone portion 12, when the head abuts the exterior of bone portion 12, the head 120,220 would be held stationary with respect to bone portion 12, whilst the leading end of screw 100,200 would travel further into bone portion 14. This would pull the bone portions 12, 14 together, compressing the fracture, as opposed to maintaining or distracting it.

It would be possible to have a radially-extending head and to avoid the above problem of compression, by using an alternative embodiment (not shown) wherein the screw 100,200 is long enough for the head not to abut the outer surface of bone portion 12 when the rest of the screw is correctly positioned in bone portions 12 and 14; this embodiment would typically not be fully inserted into the bone portions.

The greater the pitch of the screw threads, the greater the distance the screw will advance into the bone per rotation of the screw. Therefore, if a screw has screw threads of different pitches on different parts of the screw, these different parts of the screw will travel at different rates. The increase in pitch of the screw threads 112, 230, 240 towards the head 120, 220 means that for every rotation of the screw 100, 200, the tip of the screw will progress more slowly through the bone than the head 120, 220. Therefore, the leading end of the screw 100, 200, with its low-pitch screw threads will advance more slowly through bone portion 14, compared to the trailing (head) end of the screw 100, 200 advancing through bone portion 12. This will result in the two bone portions 12, 14 being pushed apart, and the screw 100, 200 being put into compression between the two bone portions. The more the screw is rotated, the more the bone portions 12, 14 are pushed apart to distract the fracture.

For example, in the case of screw 200, if the pitch of the screw threads 245 is twice the pitch of the screw threads 235, the screw threads 245 will advance into the bone twice as quickly as screw threads 235, so for every 1mm of axial movement of the leading portion 230, there will be 2mms of axial movement of the trailing portion 240 and therefore the bone portions 12, 14 will have become pushed apart (distracted) by 1mm.

The screw 100, 200 is driven through the bone until the bone portions have been distracted sufficiently. The screw 100, 200 can now be left in the bone in that position until the fracture has healed, or permanently as clinically indicated.

Maintaining the position of the bone portions is advantageous, especially for fractures such as that shown in Fig 1, where the lower side of the fracture is a clean break, and the other side is broken into multiple fragments. If the relative positions of the main bone portions are not maintained by the fixing device, and they are instead pulled together, the bone portions could pivot around the clean break, displace the comminuted fragments in the space 16, and heal out of alignment.

Distraction of the fracture (i.e. loading the bone portions to push the bone portions apart relative to each other) has been discovered to be desirable, as putting the bone portions in tension causes the bone portions to grip the formations (e.g. screw threads) of the fixing device more firmly, which reduces the likelihood of the fixing device working itself loose. Distracting the fracture loads the threads of the screw so that the screw is under compression and the threads bite more effectively into the bone.

Referring now to Figs 7 to 12, there are shown screws having modified thread profiles. Fig 7b shows an enlarged cross-sectional view of a single screw thread of Figs 7a and 8. Similarly, Fig 9b shows an enlarged cross-sectional view of a single screw thread of Figs 9a and 10. Fig 11b shows an enlarged cross-sectional view of a single screw thread of Figs 11a and 12.

In the screws of Figs 7 and 8, both the leading and trailing sides of the thread slope between the radially outer tip of the thread and the root; the slope is very steep. The steeper the slope (i.e. the closer to perpendicular with the root axis) the more difficult it is to remove the screw from the bone by an axial force. This is because the near-perpendicular surfaces provide an abrupt resistance to the passage of the screw through the bone, as compared to more gradually sloping surfaces, where the screw would use the gradual slope to ease its way through the bone. Therefore the screws of Figs 7 and 8 would be very difficult to remove from the bone, in either axial direction.

The Fig 7 screw threads vary in pitch, with the pitch increasing towards the trailing end (head end). The pitch could vary uniformly, or abruptly. Thus, the Fig 7 screw is suitable for distracting a fracture.

The Fig 8 screw has two portions of different root and outer thread diameter, wherein the thread pitch on the leading portion is smaller than that of the trailing portion. The pitch of the threads is typically in the range 1mm to 2mm at the leading (tip) end, and 2mm to 4mm at the trailing (head) end. The Fig 8 screw will typically cause a relative distraction of bone portions 12, 14 of 1mm to 2mm per rotation.

The screws of Figs 9 and 10 exhibit an alternative design of screw thread, wherein the threads have sides that are substantially perpendicular to the body. The substantially perpendicular sides are shown particularly well in Fig 9b. Like the design of threads of Figs 7 and 8, a screw with these threads would be very difficult to remove from a bone by axial force in either direction.

The Fig 9 screw has a constant root diameter, a constant outer thread diameter, but a varying pitch of thread. The Fig 9 screw comprises two discrete portions of different pitch of thread; however, alternative embodiments could have a uniformly varying pitch. The pitch of the threads is typically in the range 1mm to 2mm at the leading (tip) end, and 2mm to 4mm at the trailing (head) end. The Fig 9 screw will typically cause a relative distraction of bone portions 12, 14 of 1mm to 2mm per rotation.

The Fig 10 screw has a constant root diameter, a varying thread pitch, and a varying outer diameter of the thread. The Fig 10 screw comprises two discrete portions of different thread pitch and outer thread diameter; however, alternative embodiments could have a uniformly varying pitch/outer diameter. Alternative embodiments also having a varying root diameter could also be used. The pitch of the Fig 10 threads is typically in the range 1mm to 2mm at the leading (tip) end, and 2mm to 4mm at the trailing (head) end. The Fig 10 screw will typically cause a relative distraction of bone portions 12, 14 of 1mm to 2mm per rotation.

The screws of Figs 11 and 12 show a further alternative design of screw thread. Each thread has one perpendicular side and one sloping side; the threads at the leading end of the screw have perpendicular leading sides, and the threads at the trailing end of the screw have perpendicular trailing sides. The perpendicular sides are shown particularly well in Fig 11b.

The Fig 11 screw has threads of varying pitch. The leading and trailing ends of the Fig 11 screw have threads of a constant outer diameter and a constant root diameter. The Fig 11 screw comprises two discrete portions of different thread pitch; however, alternative embodiments could have a uniformly varying pitch. Alternative embodiments also having a varying root diameter could also be used.

The Fig 12 screw has a constant root diameter, a varying thread pitch, and a varying outer diameter of the thread. The Fig 12 screw comprises two discrete portions of different thread pitch and outer thread diameter; however, alternative embodiments could have a uniformly varying pitch/outer diameter. Alternative embodiments also having a varying root diameter could also be used. The pitch of the Fig 12 threads is typically in the range 1mm to 2mm at the leading (tip) end, and 2mm to 4mm at the trailing (head) end. The Fig 12 screw will typically cause a relative distraction of bone portions 12, 14 of 1mm to 2mm per rotation.

As explained above, the steeper the angle of the thread, the greater the resistance of the screw to removal in the direction of that edge. Steeply sloping thread edges with respect to the root (like those of the Figs 9 and 10 embodiments) will provide an abrupt resistance to axial movement, as compared to more smoothly sloping edges, which would ease the axial movement of the screw using the principal of the inclined plane. Therefore, the threads at the leading end will provide a high resistance to movement in the direction of insertion, due to the perpendicular leading edges. The threads at the trailing end will provide a high resistance to axial movement in the direction opposite to the direction of insertion, due to their perpendicular trailing edges. Thus, the screws of Figs 11 and 12 resist removal in both axial directions, due to the leading end threads resisting axial movement in the direction of insertion, and the trailing end threads resisting axial movement in the opposite direction.

The screws of Figs 7 and 12 all provide a high resistance to axial force once in the bone, due to having at least some steep edges relative to the axis of the screw in both axial directions. This is particularly useful for use in fragile bones, for example in the elderly, where the bone is so soft that a conventional screw could more easily be pulled through the bone.

All the screws of Figs 7 to 12 are suitable for distracting a fracture, as they all have screw threads of a smaller pitch at the leading end as compared to the trailing end.

It should be noted that it is not only the Fig 5 or Fig 6 design (varying root diameter) that has the advantage that the trailing threads cut into previously uncut bone. The embodiments having a constant root diameter but increased outer diameter of the threads at the trailing end (such as those shown in Figs 8, 10 and 12) also have this advantage. The larger diameter threads at the trailing end cut into previously uncut bone to secure the screw more firmly in the bone.

The bone fixing devices of all embodiments may be metallic or non-metallic, and the invention is not limited to the use of any particular material. The bone fixing device may be made of a bioabsorbable material. Examples of suitable bioabsorbable materials include polylactic acids and polyglycolic acids; however any other medically recognised bioabsorbable material could be used.

The fixing device may or may not be provided with a coating. A possible example of a suitable coating is hydroxyapatite. This can be applied to the fixing device by dipping the fixing device in the coating and allowing the coating to dry. Hydroxyapatite is a common calcium salt that is also found naturally in bone. The presence of a hydroxyapatite coating on the fixing device encourages the surrounding bone to grow, both on to the fixing device itself and, generally, in the vicinity of the screw.

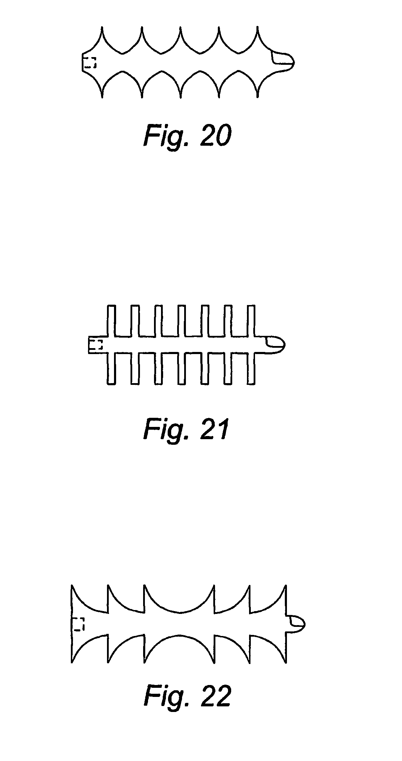

In some useful versions of the fixing device, the threads need not vary between the ends of the device, and figs 20-22 show embodiments that maintain the loading on the screw by means of the shapes of the threads, rather than by means of the changes in pitch. Despite the lack of change in pitch of the thread which can be used for active loading of the threads in a distracted fracture, the embodiments of Figs 20-22 will be subjected to loading by the natural bias of the bones tending to compress the device, which will thereby exert a loading force on the threads of the screw tending to resist dislodgement of the screw when implanted. Referring now to Fig 20, there is shown a screw having modified thread profiles similar to that shown in Fig 7, although the Fig 20 screw has a constant root diameter, a constant pitch thread and a constant outer thread diameter.

In the Fig 20 screw, both the leading and trailing sides of the thread slope between the radially outer tip of the thread and the root; the slope is very steep, and is typically non-linear, in that the slope is more gradual near the root of the screw, and steeper nearer to the radial edge of the thread. The steeper the slope (i.e. the closer to perpendicular with the root axis) the more difficult it is to remove the screw from the bone by an axial force. This is because the near-perpendicular surfaces provide an abrupt resistance to the passage of the screw through the bone, as compared to more gradually sloping surfaces, where the screw would use the gradual slope to ease its way through the bone. Therefore the Fig 20 screw would be very difficult to remove from the bone, in either axial direction.

Fig 21 is another version of a screw with rectangular threads similar to the Fig 9 embodiment, but having a constant root diameter, a constant pitch thread and a constant outer thread diameter. This screw is suitable for maintaining a fracture.

Fig 22 shows a further version of screw with a thread profile similar to that of the Fig 11 embodiment, but having a constant root diameter, a constant pitch thread and a constant outer thread diameter. This screw is suitable for maintaining a fracture.

Modifications and improvements may be incorporated without departing from the scope of the invention. For example, any of the screws shown in Figs 7 to 12 could optionally have a non-threaded mid-portion (not shown), and/or a varying root diameter (not shown).

Screw 200 and the screws of Figs 8, 10 and 12 all have an abrupt change of diameter; however, the diameter change could alternatively be uniform, such as in screw 100. Any of the designs of screw threads shown in Figs 7 to 12 could be combined with any of screws 100 and 200.

All of the screws in Figs 7 to 12 can have self-drilling, self-tapping tips; they may be cannulated; they may have non-threaded mid-portions; and they may have a head which does not project outwardly of the surface of the root.

Although the examples here are described with reference to wrist fractures, the invention could be used for any fracture.

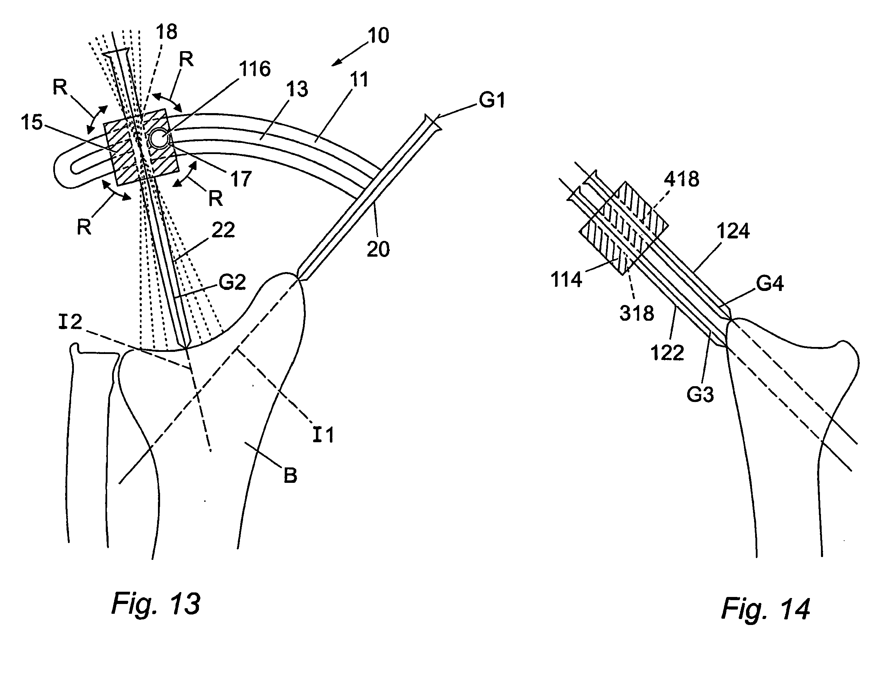

Fig 13 shows a jig 10 which is being used to define two lines of insertion of two threaded bone fixing devices (not shown) into a bone B. In this example, bone B is a radius, and the fixing devices are to be inserted in the head of the radius. However, the invention is not limited to the use in any particular part of any particular bone.

The head of bone B is typically fractured (fracture lines not shown), and the fixing devices (e.g. bone screws) are to be inserted to hold the fracture fragments together.

The jig 10 has an elongate arm 11 which has the form of an arc. The arm 11 has a slot 13 which extends along the length of the arm 11. The slot 13 extends all the way through the arm 11.

A first sleeve 20 is coupled to one of the elongate ends of the arm 11 such that the arm 11 extends from a lateral side of the sleeve 20. The first sleeve 20 may be coupled to the arm 11 by any suitable means. Preferably, the sleeve 20 is slidably mounted on the arm, for example the arm 11 may have a guide slot and the sleeve 20 may be slidably mounted in the slot. The sleeve 20 has an inner bore which is adapted to receive a first guide wire G1. The first sleeve 20 may be a standard guide/drill sleeve, but any guide means (typically with an aperture to receive the guide wire or bone fixing device) can be used.

A spacer 15 in the form of an alignment block 15 is coupled to the arm 11 by a bolt 116. The spacer 15 is cuboid, and has a bore 17 in which the bolt 116 is received. The bolt 116 also passes through the slot 13. A nut (not shown) engages one end of the bolt 116 to hold the spacer 15 fixed relative to the arm 11.

When the nut is loosened, the bolt 116 can move within the slot 13 so that the spacer 15 moves relative to the slot 13; the spacer 15 can also be rotated around the axis of the bolt 116 in the plane defined by the arcuate arm 11. This rotation is shown in Fig 13 by the arrows R.

The spacer 15 also has a further bore 18, which extends through the spacer 15 in a direction perpendicular to the bore 17. The bore 18 goes directly between opposite sides of the cuboid spacer 15 in a straight line which is perpendicular to the opposite sides (not at an inclined angle through the block). Therefore, the bore 18 lies in a plane parallel to the plane of the elongate arm 11. The bore 18 is adapted to receive a second sleeve 22. Preferably, the second sleeve 22 is slidably mountable and optionally rotatably mountable in the bore 18. The second sleeve 22 is similar to the first sleeve 20, and has an inner bore adapted to receive a second guide wire G2.

Although just single sleeves 20, 22 are shown in Fig 13, each of these can represent a set of concentric sleeves. For example, sleeve 22 can represent an inner sleeve for a guide wire, an intermediate sleeve for a drill and an outer sleeve for a fixing device. The same applies to sleeve 20.

The second sleeve 22 defines a second line of insertion 12 and the first sleeve 20 defines a first line of insertion 11. The lines of insertion I1, I2 are continuations of the axes of the sleeves 20, 22 into the bone.

The lines of insertion I1, I2 lie in parallel planes, because their relative positions are defined by the sleeves 20, 22, which are in turn defined by the jig 10. The first line of insertion I1 always lies in the plane of the elongate arm 11, as the first sleeve 20 is fixed (apart from any slidable mounting) to the end of the arm 11, the arm 11 extending from a lateral side of the first sleeve 20.

As explained above, the bore 18 lies in a plane parallel to the plane of the elongate arm 11; therefore the second line of insertion I2 always lies in a plane parallel to the plane of the elongate arm 11. This is always true independent of the selected rotational and translational position of the spacer 15. In the Fig 13 view, the plane of the second line of insertion I2 is in front of the plane of the elongate arm 11.

The second line of insertion I2 is adjustable relative to the first line of insertion I1 by altering the rotational and translational position of the spacer 15. The angles of the guide sleeves 20, 22 can therefore be adjusted so that the lines of insertion I1, I2 cross when viewed in a direction perpendicular to the parallel planes of the lines of insertion I1, I2 (as seen in the Fig 13 view).

Because the arm 11 is arcuate (see Fig 13) no additional rotational adjustment of the spacer 15 relative to the slot 13 is necessary in order to ensure that the lines of insertion cross. For example, in some embodiments, rotation of the spacer is guided by the arcuate slot 13 so that on moving (translating) the spacer 15 along the slot 13, the spacer 15 is automatically rotated so that the axis of the bore 18 is always perpendicular to the part of the slot 13 in the vicinity of the spacer 15. This could be achieved by providing the spacer 15 with one or more lateral projections adapted to engage the slot 13. These embodiments can provide jigs which are simple to operate, since a change in translational position of the spacer along the arcuate jig automatically adjusts the spacer's rotational position so that the respective lines of insertion cross in the desired location when seen from the Fig 13 direction. However, the capability to rotate the spacer 15 is advantageous, as this allows a very precise selection of the angle of insertion (several alternative options for the second line of insertion are shown in dotted lines in Fig 13).

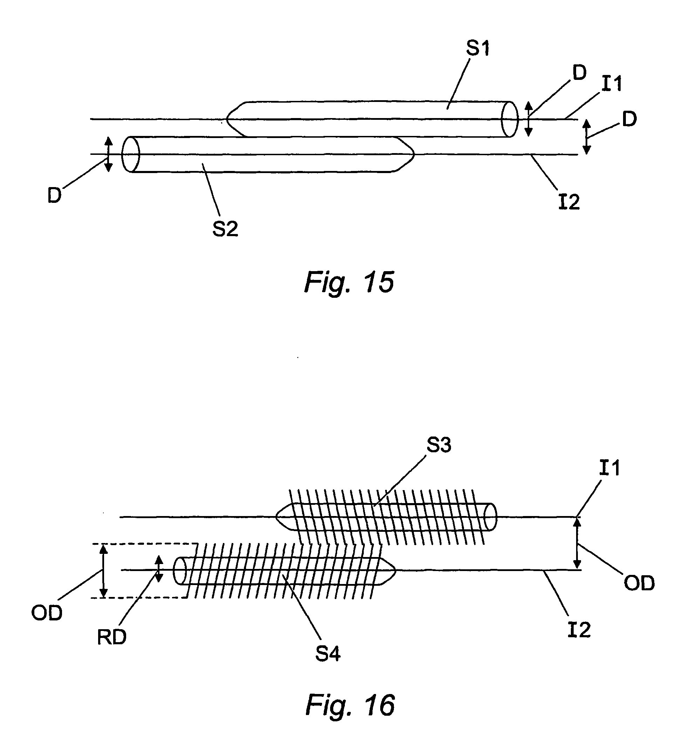

The position of the bore 18 in the spacer 15 is selected such that the distance between the plane of the elongate arm 11 (the plane of the first line of insertion) and the parallel plane in which the bore 18 lies (the plane of the second line of insertion) is greater than or equal to the diameter of the bone fixing device (the outer thread diameter, if threaded). The reason for this will be explained with reference to Figs 15 and 16. In Fig 15, D represents the diameter of the fixing devices, and in Fig 16, RD represents the root diameter of the fixing devices, and OD represents the outer (thread) diameter of the fixing devices.

Fig 15 shows a view from above of two non-threaded bone fixing devices S1, S2 which have been inserted along respective lines of insertion I1, I2. The tips of the fixing devices and the lines of insertion I1, I2 are angled into the page. The lines I1, I2 also represent the positions of the parallel planes of the lines of insertion in this view. In Fig 15, the distance between the parallel planes is equal to the diameter D; therefore the fixing devices will just touch, but they will not collide.

Fig 16 shows a further embodiment where the bone fixing devices S3, S4 are threaded. In this embodiment, the distance between the parallel planes is equal to the outer (thread) diameter OD, and the outer threads of the inserted fixing devices will just touch but not interlock.

Both Figs 15 and 16 show how two bone fixing devices can be inserted into positions where they just touch but do not collide.

This is advantageous, as one fixing device is able to lean against the other; one fixing device effectively buttressing the other. This provides additional support for the bone fixing devices, which helps to keep them in the bone. In the threaded embodiments this support is additional to the friction between the threads and the bone. The fixing devices do not have to be literally touching each other to achieve this effect; bone fixing devices a small distance apart could also achieve this effect. The invention encompasses both literally touching embodiments and other embodiments in which the bone fixing devices pass close together.

Fig 15 illustrates that the bone fixing devices are not necessarily threaded, and can be simple rods.

In use, the position of the first line of insertion is selected and a first guide wire G1 is inserted (e.g. using a guide sleeve) to define the first line of insertion I1. Next, a hole is drilled for the first fixing device using a cannulated drill (and also typically a drill sleeve). Next, a first fixing device is inserted into the bone over the guide wire G1 into the hole drilled by the drill (optionally using a tap if the fixing device is not self-tapping). In this method, typically an appropriate sized guide/drill sleeve is selected for each step.

Next, the guide sleeves 20, 22 are coupled to the jig 10 and this assembly is then positioned as shown in Fig 13, with the first sleeve 20 being inserted over the protruding part of the guide wire G1. A screwdriver may be held in the first sleeve 20 so that it engages the first fixing device during the subsequent steps, to provide additional stability to the assembly whilst the second line of insertion is defined.

Next the nut (not shown) is loosened and the spacer is moved relative to the slot 13 to a desired translational and rotational position so that the second guide sleeve 22 defines a second line of insertion I2.

The surgeon typically ensures that the second line of insertion I2 crosses the first line of insertion I1 when viewed in a direction perpendicular to the parallel planes of the lines of insertion I1, I2 (i.e. in the direction of the front view of Fig 13).

Once the second line of insertion I2 has been determined, the nut is tightened against the bolt to fix the rotational and translational position of the second sleeve 22. A second guide wire G2 is inserted through the second sleeve 22 and into the bone along the second line of insertion I2.

As explained above, the second sleeve 22 can represent a set of concentric sleeves, and the second guide wire G2 is typically inserted through an inner sleeve. Next, the inner sleeve can be removed, and a hole is drilled in the bone using a cannulated drill inserted over the second guide wire G2 using an intermediate-sized sleeve to guide the drill. Next, that sleeve is removed and an outer sleeve is used to guide the insertion of the second fixing device into the bone. If the fixing device is not self-tapping, a tap can be used to create space for a thread in the bone before insertion of the second fixing device; the tap can also be guided by the outer sleeve.

The bone fixing devices are typically cannulated bone screws S3, S4 and the guide wires G1, G2 guide the paths of the bone fixing devices in the bone. Because the lines of insertion have been accurately defined by the jig 10, the fixing devices S3, S4 can be positioned such that they pass closely together, without colliding (optionally even touching each other). This provides additional support to prevent the fixing devices S3, S4 coming loose and falling out of the bone. One fixing device effectively buttresses the other, which strengthens the connection of both fixing devices to the bone.

In a slightly modified method, the jig 10 and guide sleeves 20, 22 can be used to insert the first guide wire G1 and the first fixing device into the bone, instead of the jig 10 first being used after these have been inserted.

In some versions of the method, the jig 10 and sleeve 20 may be used to insert the first fixing device but not the first guide wire G1.

In all methods, it is advantageous to use some sort of guide sleeve (whether or not attached to the jig 10) to aid insertion of the guide wires, drill and fixing devices, to protect the surrounding tissues.

Fig 14 shows an alternative embodiment of the invention, where the spacer 114 is modified to include more than one guide bore. In this embodiment, two parallel guide bores, 318, 418 are provided. The guide bores 318, 418 are adapted to receive respective sleeves 122, 124, which define lines of insertion of respective guide wires G3, G4. Because the guide bores 318, 418 are parallel, the lines of insertion they defined will not intersect and screws inserted through sleeves 122, 124 will not collide with each other. The guide bores 318, 418 may both lie in a single plane parallel to the plane of the arm 11 (i.e. side by side in the Fig 13 view), or the guide bores 122, 124 may each lie in different planes parallel to the plane of the arm 11 (i.e. on in front of the other in the Fig 13 view). The Fig 14 embodiment may be particularly useful when the bone has fractured into many fragments, as including an extra fixing device can help to ensure that more of the fracture fragments are held together.

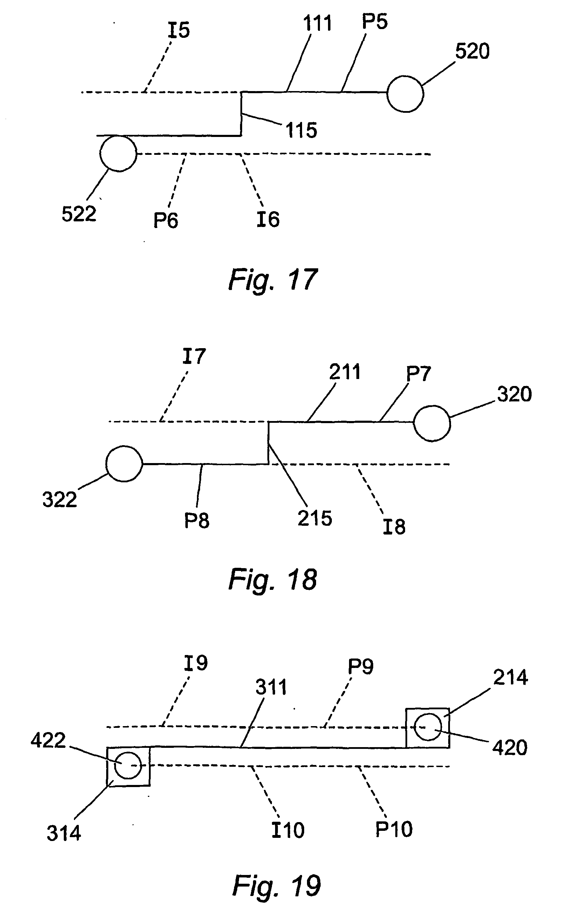

Figs 17 to 19 show alternative embodiments of the invention. Fig 17 shows a modified arm 111 which includes a stepped portion 115. The stepped portion 115 divides the arm 111 into two portions which lie in parallel planes P5, P6; the stepped portion is an intermediate part of the arm 111 which is perpendicular to both portions of the arm 111. When seen from the front (like the Fig 13 view), the P6 plane would be in front of the P5 plane. Therefore in this embodiment, the stepped arm 111 functions as a spacer, creating two parallel planes without the need for a separate spacer component 114.

First and second sleeves 520, 522 are attached to the arm 111. Only the top part of the sleeves 520, 522 are shown for clarity, but the sleeves actually extend downwards into the paper and towards each other, along the lines of insertion I5, I6. The first sleeve 520 is attached to the arm 111 so that the arm 111 extends from a lateral side of the sleeve 520 (similarly to the Fig 13 embodiment). Therefore, the first sleeve defines a line of insertion I5 which lies in a plane P5 which is coplanar with the plane its end of the arm 111.

The second sleeve 522 is attached to a lateral side of the arm 111. The second sleeve 522 may be clamped onto the arm 111 by a clamp (not shown) that extends around the end of the arm 111. The second sleeve 522 may be rotatably and/or slidably mounted on the arm 111, or alternatively it may be fixed relative to the arm 111. Since the second sleeve 522 is attached to a lateral side of the arm 111, the line of insertion I6 defined by the sleeve 522 will lie in a plane P6 which is in front of the plane P6 of that end of the arm 111 (when viewed from the front). However, the line of insertion I6 is parallel to the plane P6. Therefore, the lines of insertion I5, I6 lie in respectively parallel planes.

Fig 18 shows a further embodiment having an arm 211 with a stepped portion 215 defining two arm portions in parallel planes P7, P8, and first and second sleeves 320, 322 attached (e.g. slidably mounted) to the arm. This embodiment is the same as the Fig 17 embodiment in most respects, except that the second sleeve 322 is attached to the arm 211 so that the arm 211 extends from a lateral side of the second sleeve 322. Therefore, in this embodiment, the lines of insertion I7, I8 defined by the sleeves 320, 322 will both lie along the parallel planes P7, P8 defined by the arm 211. The first and second sleeves 320, 322 are not rotatably mounted on the arm 111. However, in alternative embodiments, one or both of the first and second sleeves 320, 322 are rotatably mounted.

Fig 19 shows a further embodiment of a jig having a non-stepped arm 311. The arm 311 is provided with two spacers 214, 314, which may be substantially the same as either of the spacers 15, 114 described above. The spacers 214, 314 are provided with respective sleeves 420, 422. In this view, the spacers 214, 314 are located at or near opposite ends of the arm, but one or both spacers is preferably translatable along the length of the arm 311 (e.g. by providing a slot as shown in the Fig 13 embodiment).

The spacers 214, 314 are provided on opposite planar faces of the arm 311, and define two lines of insertion I9, I10, both of which lie in mutually parallel planes P9, P10; these planes are also parallel to the arm 311. At least one of the spacers 214, 314 is rotatable relative to the arm 311, so that the lines of insertion I9, I10 can be made to cross when viewed in a direction perpendicular to the parallel planes P9, P10. Thus, in this embodiment, the two spacers 214, 314 together define the spacing of the parallel planes.

In some embodiments, a stepped arm and a separate spacer component can both be used in conjunction to provide the two parallel planes of the lines of insertion.

The arms 111, 211, 311 could be either straight or arcuate when viewed from a front view perpendicular to the top view shown in these figures.

Modifications and improvements may be incorporated without departing from the scope of the invention. For example, the jig 10 need not be in the shape of an arc. In an alternative embodiment, the jig 10 could be straight.

In some embodiments, the first guide sleeve could be rotatably mounted on the arm and the second guide sleeve could be rotationally fixed with respect to the arm.

In some embodiments, the sleeves 20, 22 can comprise components of the jig 10, e.g. one or both of the sleeves may be permanently attached to the jig 10. In other embodiments, the sleeves 20, 22 are attachable to and removable from the jig 10 (e.g. by being slidably mounted on the arm/spacer). Although not shown in Fig 13, an eye-member could be provided on the end of the arm 11, so that the first sleeve 20 can be slidably mounted in the eye member.

In further alternative embodiments, the arm could be extendible. An extendible arm could provide an alternative to a slotted arm, as this would also enable relative translation of the two guide sleeves. In some embodiments, the arm may be both slotted and extendible.

In further alternative embodiments, the jig is adapted to allow insertion of bone fixing devices at locations spaced apart by significant distances. Such embodiments provide an increased choice of relative angles and positions of the two fixing devices. In these embodiments, the fixing devices do not necessarily touch or even come close to each other, and the surgeon can still be confident that no collision will occur.

As illustrated in Fig 15, the bone fixing devices are not necessarily threaded, and can be rods.

## Claims

1. An assembly comprising:
a first bone fixing device (S1); and
a jig (10) comprising first and second guide means (20, 22), the position of the second guide means (22) being variable relative to the position of the first guide means (20);
**characterised in that**:
the assembly also includes a second bone fixing device (S2);
the jig is for positioning the first and second bone fixing devices (S1, S2) into an area of fractured bone;
the first and second guide means (20, 22) define first and second lines of insertion (I1, I2) for the first and second bone fixing devices (S1, S2) respectively;
the lines of insertion (I1, I2) lie in parallel planes and the lines of insertion (I1, I2) cross when viewed in a direction perpendicular to the parallel planes; and
the parallel planes have a separation greater than or equal to the diameter (D) of the bone fixing devices (S1, S2).

2. An assembly as claimed in claim 1, wherein the jig (10) defines more than two lines of insertion.

3. An assembly as claimed in claim 1 or claim 2, wherein the orientation of the second guide means (22) is variable relative to the orientation of the first guide means (20).

4. An assembly as claimed in any preceding claim, wherein the second guide means (22) is translatable relative to the first guide means (2Q).

5. An assembly as claimed in any preceding claim, wherein the first and second guide means (20, 22) comprise first and second guide sleeves.

6. An assembly as claimed in any preceding claim, wherein the first and second guide means (20, 22) are adapted to receive guide wires and/or the bone fixing devices (S1, S2).

7. An assembly as claimed in any preceding claim, wherein the jig (10) includes an arm (11) that extends in a lateral direction with respect to at least one of the lines of insertion (I1, I2).

8. An assembly as claimed in claim 7, wherein at least a part of the arm (11) has a planar surface that is parallel to the parallel planes of the lines of insertion (I1, I2).

9. An assembly as claimed in claim 8, wherein at least a part of the arm (11) has a planar surface that is co-planar with one of the parallel planes of the lines of insertion (I1, I2).

10. An assembly as claimed in any of claims. 7 to 9, wherein the first and second guide means (20, 22) are coupled to the arm (11).

11. An assembly as claimed in any of claims 7 to 10, wherein the arm (11) is arcuate.

12. An assembly as claimed in any of claims 7 to 11, wherein the jig (10) includes a spacer (15) adapted to space the parallel planes of the lines of insertion (I1, I2) apart from each other.

13. An assembly as claimed in claim 12, wherein the spacer (15) is an individual component distinct from the arm (11).

14. An assembly as claimed in claim 12 or claim 13, wherein the spacer (15) comprises a block having a guide bore (18) adapted to receive a guide sleeve (22).

15. An assembly as claimed in any of claims 12 to 14, wherein the spacer (15) has an attachment bore (17) and the spacer (15) is coupled to the arm (11) by an attachment device (116) inserted through the attachment bore (17) in the spacer (15).

16. An assembly as claimed in any of claims 12 to 15, wherein the spacer (15) is rotatably and/or translatably mounted on the arm (11).

17. An assembly as claimed in any of claims 12 to 16, wherein an elongate aperture is provided in the arm (11) and the spacer (15) is translatable along the elongate aperture.

18. An assembly as claimed in claim 17, wherein the elongate aperture is arcuate, and wherein the spacer (15) is rotationally coupled to the elongate aperture, such that as the spacer (15) translates along the aperture, the spacer (15) rotates with the curvature of the aperture.

19. An assembly as claimed in claim 18 when dependent on claim 14, wherein on translation of the spacer (15), the spacer (15) rotates such that the guide bore (18) is always perpendicular to a tangent to the elongate aperture at the location of the spacer (15).

20. An assembly as claimed in any of claims 12 to 19, wherein the position of one of the guide means (20, 22) is defined by the arm (11) and the position of the other guide means (20, 22) is defined by the spacer (15), such that moving the spacer (15) in rotation and/or translation relative to the arm (11) varies the orientation/spacing of the first and second guide means (20, 22) relative to each other.

21. An assembly as claimed in any one of claims 12 to 20, wherein the arm (11) comprises a first portion having a first planar surface and a second portion having a second planar surface aligned parallel to the first planar surface, and wherein the second portion is stepped relative to the first portion.

22. An assembly as claimed in claim 12, wherein the spacer (115) comprises a part of the arm (111).

23. An assembly as claimed in claim 22, wherein the arm (111) comprises a first portion having a first planar surface and a second portion having a second planar surface aligned parallel to the first planar surface, and wherein the second portion is stepped relative to the first portion.

24. An assembly as claimed in claim 22 or claim 23, wherein at least one of the first and second guide means (520, 522) is rotatably and/or translatably mounted on the arm (111).

25. An assembly as claimed in any of claims 7 to 24, wherein the arm (11) is extendible.

26. An assembly as claimed in any preceding claim, wherein the bone fixing devices (S1, S2) are at least partially threaded.

## Patentansprüche

1. Eine Anordnung, die Folgendes beinhaltet:
eine erste Knochenfixiervorrichtung (S1) und
eine Bohrlehre (10), die ein erstes und ein zweites Führungsmittel (20, 22) beinhaltet, wobei die Position des zweiten Führungsmittels (22) relativ zu der Position des ersten Führungsmittels (20) variabel ist;
**dadurch gekennzeichnet, dass**
die Anordnung auch eine zweite Knochenfixiervorrichtung (S2) umfasst;
die Bohrlehre zum Positionieren der ersten und der zweiten Knochenfixiervorrichtung (S1, S2) in einen Bereich des gebrochenen Knochens dient;
das erste und das zweite Führungsmittel (20, 22) eine erste und eine zweite Einführungslinie (I1, I2) für die erste bzw. die zweite Knochenfixiervorrichtung (S1, S2) definieren;
die Einführungslinien (I1, I2) in parallelen Ebenen liegen und sich die Einführungslinien (I1, I2) kreuzen, wenn in einer zu den parallelen Ebenen senkrechten Richtung betrachtet; und
die parallelen Ebenen eine Trennung aufweisen, die größer als oder gleich dem Durchmesser (D) der Knochenfixiervorrichtungen (S1, S2) ist.

2. Anordnung gemäß Anspruch 1, wobei die Bohrlehre (10) mehr als zwei Einführungslinien definiert.

3. Anordnung gemäß Anspruch 1 oder Anspruch 2, wobei die Ausrichtung des zweiten Führungsmittels (22) relativ zu der Ausrichtung des ersten Führungsmittels (20) variabel ist.

4. Anordnung gemäß einem der vorhergehenden Ansprüche, wobei das zweite Führungsmittel (22) relativ zu dem ersten Führungsmittel (20) verschiebbar ist.

5. Anordnung gemäß einem der vorhergehenden Ansprüche, wobei das erste und das zweite Führungsmittel (20, 22) eine erste und eine zweite Führungshülse beinhalten.

6. Anordnung gemäß einem der vorhergehenden Ansprüche, wobei das erste und das zweite Führungsmittel (20, 22) angepasst sind, um Führungsdrähte und/oder Knochenfixiervorrichtungen (S1, S2) aufzunehmen.

7. Anordnung gemäß einem der vorhergehenden Ansprüche, wobei die Bohrlehre (10) einen Arm (11) umfasst, der sich mit Bezug auf mindestens eine der Einführungslinien (I1, I2) in einer lateralen Richtung erstreckt.

8. Anordnung gemäß Anspruch 7, wobei mindestens ein Teil des Arms (11) eine planare Oberfläche aufweist, die zu den parallelen Ebenen der Einführungslinien (I1, I2) parallel ist.

9. Anordnung gemäß Anspruch 8, wobei mindestens ein Teil des Arms (11) eine planare Oberfläche aufweist, die zu einer der parallelen Ebenen der Einführungslinien (I1, I2) koplanar ist.

10. Anordnung gemäß einem der Ansprüche 7 bis 9, wobei das erste und das zweite Führungsmittel (20, 22) an den Arm (11) gekoppelt sind.

11. Anordnung gemäß einem der Ansprüche 7 bis 10, wobei der Arm (11) bogenförmig ist.

12. Anordnung gemäß einem der Ansprüche 7 bis 11, wobei die Bohrlehre (10) einen Abstandshalter (15) umfasst, der angepasst ist, um die parallelen Ebenen der Einführungslinien (I1, I2) mit Abstand zueinander anzuordnen.

13. Anordnung gemäß Anspruch 12, wobei der Abstandshalter (15) eine sich von dem Arm (11) unterscheidende individuelle Komponente ist.

14. Anordnung gemäß Anspruch 12 oder Anspruch 13, wobei der Abstandshalter (15) einen Block mit einer Führungsbohrung (18), die angepasst ist, um eine Führungshülse (22) aufzunehmen, beinhaltet.

15. Anordnung gemäß einem der Ansprüche 12 bis 14, wobei der Abstandshalter (15) eine Anbringungsbohrung (17) aufweist und der Abstandshalter (15) mittels einer Anbringungsvorrichtung (116), die durch die Anbringungsbohrung (17) in dem Abstandshalter (15) eingeführt wird, an den Arm (11) gekoppelt ist.

16. Anordnung gemäß einem der Ansprüche 12 bis 15, wobei der Abstandshalter (15) rotierbar und/oder verschiebbar an dem Arm (11) montiert ist.

17. Anordnung gemäß einem der Ansprüche 12 bis 16, wobei in dem Arm (11) eine längliche Öffnung bereitgestellt ist und der Abstandshalter (15) entlang der länglichen Öffnung verschiebbar ist.

18. Anordnung gemäß Anspruch 17, wobei die längliche Öffnung bogenförmig ist und wobei der Abstandshalter (15) rotierbar an die längliche Öffnung gekoppelt ist, so dass, wenn sich der Abstandshalter (15) entlang der Öffnung verschiebt, der Abstandshalter (15) mit der Bogenlinie der Öffnung rotiert.

19. Anordnung gemäß Anspruch 18, wenn von Anspruch 14 abhängig, wobei bei Verschiebung des Abstandshalters (15) der Abstandshalter (15) so rotiert, dass die Führungsbohrung (18) zu einer Tangente an die längliche Öffnung an der Stelle des Abstandshalters (15) immer senkrecht ist.

20. Anordnung gemäß einem der Ansprüche 12 bis 19, wobei die Position von einem der Führungsmittel (20, 22) von dem Arm (11) definiert wird und die Position von dem anderen Führungsmittel (20, 22) von dem Abstandshalter (15) definiert wird, so dass das Bewegen des Abstandshalters (15) als Rotieren und/oder Verschieben relativ zu dem Arm (11) die Ausrichtung/den Abstand des ersten und des zweiten Führungsmittels (20, 22) relativ zueinander variiert.

21. Anordnung gemäß einem der Ansprüche 12 bis 20, wobei der Arm (11) einen ersten Abschnitt mit einer ersten planaren Oberfläche und einen zweiten Abschnitt mit einer zweiten planaren Oberfläche, die zu der ersten planaren Oberfläche parallel abgestimmt ist, beinhaltet und wobei der zweite Abschnitt relativ zu dem ersten Abschnitt abgestuft ist.

22. Anordnung gemäß Anspruch 12, wobei der Abstandshalter (115) einen Teil des Arms (111) beinhaltet.

23. Anordnung gemäß Anspruch 22, wobei der Arm (111) einen ersten Abschnitt mit einer ersten planaren Oberfläche und einen zweiten Abschnitt mit einer zweiten planaren Oberfläche, die zu der ersten planaren Oberfläche parallel abgestimmt ist, beinhaltet und wobei der zweite Abschnitt relativ zu dem ersten Abschnitt abgestuft ist.

24. Anordnung gemäß Anspruch 22 oder Anspruch 23, wobei mindestens eines von dem ersten und dem zweiten Führungsmittel (520, 522) rotierbar und/oder verschiebbar an dem Arm (111) montiert ist.

25. Anordnung gemäß einem der Ansprüche 7 bis 24, wobei der Arm (11) erweiterbar ist.

26. Anordnung gemäß einem der vorhergehenden Ansprüche, wobei die Knochenfixiervorrichtungen (S1, S2) mindestens teilweise mit einem Gewinde versehen sind.

## Revendications

1. Un assemblage comprenant :
un premier dispositif de fixation d'os (S1) ; et
un gabarit (10) comprenant des premier et deuxième moyens de guidage (20, 22),
la position du deuxième moyen de guidage (22) étant variable par rapport à la position du premier moyen de guidage (20) ;
**caractérisé en ce que** :
l'assemblage inclut également un deuxième dispositif de fixation d'os (S2) ;
le gabarit est destiné à positionner les premier et deuxième dispositifs de fixation d'os (S1, S2) dans une zone d'os fracturé ;
les premier et deuxième moyens de guidage (20, 22) définissent des première et deuxième lignes d'insertion (I1, I2) pour les premier et deuxième dispositifs de fixation d'os (S1, S2) respectivement ;
les lignes d'insertion (I1, I2) se trouvent dans des plans parallèles et les lignes d'insertion (I1, I2) se croisent lorsqu'elles sont vues dans une direction perpendiculaire aux plans parallèles ; et
les plans parallèles ont une séparation supérieure ou égale au diamètre (D) des dispositifs de fixation d'os (S1, S2).

2. Un assemblage tel que revendiqué dans la revendication 1, dans lequel le gabarit (10) définit plus de deux lignes d'insertion.

3. Un assemblage tel que revendiqué dans la revendication 1 ou la revendication 2, dans lequel l'orientation du deuxième moyen de guidage (22) est variable par rapport à l'orientation du premier moyen de guidage (20).

4. Un assemblage tel que revendiqué dans n'importe quelle revendication précédente, dans lequel le deuxième moyen de guidage (22) peut être translaté par rapport au premier moyen de guidage (20).

5. Un assemblage tel que revendiqué dans n'importe quelle revendication précédente, dans lequel les premier et deuxième moyens de guidage (20, 22) comprennent des premier et deuxième manchons de guidage.

6. Un assemblage tel que revendiqué dans n'importe quelle revendication précédente, dans lequel les premier et deuxième moyens de guidage (20, 22) sont adaptés pour recevoir des fils-guides et / ou les dispositifs de fixation d'os (S1, S2).

7. Un assemblage tel que revendiqué dans n'importe quelle revendication précédente, dans lequel le gabarit (10) inclut un bras (11) qui s'étend dans une direction latérale par rapport à au moins une des lignes d'insertion (I1, I2).

8. Un assemblage tel que revendiqué dans la revendication 7, dans lequel au moins une partie du bras (11) a une surface plane qui est parallèle aux plans parallèles des lignes d'insertion (I1, I2).

9. Un assemblage tel que revendiqué dans la revendication 8, dans lequel au moins une partie du bras (11) a une surface plane qui est coplanaire avec l'un des plans parallèles des lignes d'insertion (I1, I2).

10. Un assemblage tel que revendiqué dans n'importe lesquelles des revendications 7 à 9, dans lequel les premier et deuxième moyens de guidage (20, 22) sont couplés au bras (11).

11. Un assemblage tel que revendiqué dans n'importe lesquelles des revendications 7 à 10, dans lequel le bras (11) est arqué.

12. Un assemblage tel que revendiqué dans n'importe lesquelles des revendications 7 à 11, dans lequel le gabarit (10) inclut un espaceur (15) adapté pour espacer les plans parallèles des lignes d'insertion (I1, I2) à l'écart l'un de l'autre.

13. Un assemblage tel que revendiqué dans la revendication 12, dans lequel l'espaceur (15) est un composant individuel distinct du bras (11).

14. Un assemblage tel que revendiqué dans la revendication 12 ou la revendication 13, dans lequel l'espaceur (15) comprend un bloc ayant un alésage de guidage (18) adapté pour recevoir un manchon de guidage (22).

15. Un assemblage tel que revendiqué dans n'importe lesquelles des revendications 12 à 14, dans lequel l'espaceur (15) a un alésage d'attache (17) et l'espaceur (15) est couplé au bras (11) par un dispositif d'attache (116) inséré à travers l'alésage d'attache (17) dans l'espaceur (15).

16. Un assemblage tel que revendiqué dans n'importe lesquelles des revendications 12 à 15, dans lequel l'espaceur (15) est monté de façon à pouvoir tourner et / ou effectuer une translation sur le bras (11).

17. Un assemblage tel que revendiqué dans n'importe lesquelles des revendications 12 à 16, dans lequel une ouverture allongée est fournie dans le bras (11) et l'espaceur (15) peut effectuer une translation le long de l'ouverture allongée.

18. Un assemblage tel que revendiqué dans la revendication 17, dans lequel l'ouverture allongée est arquée, et dans lequel l'espaceur (15) est couplé de façon à tourner à l'ouverture allongée de telle sorte que, alors que l'espaceur (15) effectue une translation le long de l'ouverture, l'espaceur (15) tourne avec la courbure de l'ouverture.

19. Un assemblage tel que revendiqué dans la revendication 18 lorsqu'elle dépend de la revendication 14, dans lequel lors de la translation de l'espaceur (15), l'espaceur (15) tourne de telle sorte que l'alésage de guidage (18) soit toujours perpendiculaire à une tangente à l'ouverture allongée au niveau de l'emplacement de l'espaceur (15).

20. Un assemblage tel que revendiqué dans n'importe lesquelles des revendications 12 à 19, dans lequel la position de l'un des moyens de guidage (20, 22) est définie par le bras (11) et la position de l'autre moyen de guidage (20, 22) est définie par l'espaceur (15), de telle sorte que le fait de déplacer l'espaceur (15) en rotation et / ou translation par rapport au bras (11) fait varier l'orientation / l'espacement des premier et deuxième moyens de guidage (20, 22) l'un par rapport à l'autre.

21. Un assemblage tel que revendiqué dans n'importe laquelle des revendications 12 à 20, dans lequel le bras (11) comprend une première portion ayant une première surface plane et une deuxième portion ayant une deuxième surface plane alignée de façon parallèle à la première surface plane, et dans lequel la deuxième portion est en gradin par rapport à la première portion.

22. Un assemblage tel que revendiqué dans la revendication 12, dans lequel l'espaceur (115) comprend une partie du bras (111).

23. Un assemblage tel que revendiqué dans la revendication 22, dans lequel le bras (111) comprend une première portion ayant une première surface plane et une deuxième portion ayant une deuxième surface plane alignée de façon parallèle à la première surface plane, et dans lequel la deuxième portion est en gradin par rapport à la première portion.

24. Un assemblage tel que revendiqué dans la revendication 22 ou la revendication 23, dans lequel au moins un moyen de guidage parmi les premier et deuxième moyens de guidage (520, 522) est monté de façon à pouvoir tourner et / ou de façon à pouvoir effectuer une translation sur le bras (111).

25. Un assemblage tel que revendiqué dans n'importe lesquelles des revendications 7 à 24, dans lequel le bras (11) est extensible.

26. Un assemblage tel que revendiqué dans n'importe quelle revendication précédente, dans lequel les dispositifs de fixation d'os (S1, S2) sont au moins en partie filetés.
